# FASCICULE DE BREVET EUROPEEN

(11) **EP 1 569 642 B1**
(45) Date de publication et mention de la délivrance du brevet: **11.08.2010**
(21) Numéro de dépôt: 03789474.8
(22) Date de dépôt: 24.11.2003
(51) Int. Cl.: A61K 31/4375, A61K 36/185, A61K 36/758, A61P 33/02, C07D 471/16

(54) **UTILISATION DE LA CANTHIN-6-ONE, DES EXTRAITS DE PLANTES LA CONTENANT ET DE SES DERIVES DANS LE TRAITEMENT DES TRYPANOSOMIASES**
VERWENDUNG VON CANTHIN-6-ON, VON PFLANZENEXTRAKTEN DIESES ENTHALTEND UND VON DESSEN DERIVATEN FÜR DIE BEHANDLUNG VON TRYPANOSOMIASIS
USE OF CANTHIN-6-ONE, PLANT EXTRACTS CONTAINING SAME AND DERIVATIVES THEREOF IN THE TREATMENT OF TRYPANOSOMIASES

(30) Priorité: 25.11.2002 FR 0214729
(43) Date de publication de la demande: 07.09.2005
(73) Titulaire: Institut de Recherche pour le Développement ( IRD), 75480 Paris Cedex 10 (FR); Universite Nationale d'Asuncion, CC2511 Asuncion (PY)
(72) Inventeur: FERREIRA, Maria-Elena, Lugue (PY); FOURNET, Alain, F-40590 Ossages (FR); ROJAS DE ARIAS, Antonieta, 1165 Atilio Pena (PY); HOCQUEMILLER, Reynald, F-91470 Limours (FR); POUPON, Erwan, F-92160 Antony (FR)
(74) Mandataire: Corizzi, Valérie
(86) Numéro de dépôt international: PCT/FR2003/003459
(87) Numéro de publication internationale: WO 2004/050092

(56) Documents cités:
- WO-A-03/094990
- ODEBIYI O O ET AL: "ANTIMICROBIAL ALKALOIDS FROM A NIGERIAN CHEWING STICK (FAGARA ZANTHOXYLOIDES)" PLANTA MEDICA, THIEME, STUTTGART, DE, vol. 36, no. 3, 1979, pages 204-207, XP008007203 ISSN: 0032-0943
- FERREIRA M E ET AL: "LEISHMANICIDAL ACTIVITY OF TWO CANTHIN-6-ONE ALKALOIDS, TWO MAJOR CONSTITUENTS OF ZANTHOXYLUM CHILOPERONE VAR. ANGUSTIFOLIUM" JOURNAL OF ETHNOPHARMACOLOGY, ELSEVIER SCIENTIFIC PUBLISHERS LTD, IE, vol. 80, no. 2/3, 2002, pages 199-202, XP001104505 ISSN: 0378-8741
- DIEHL E E ET AL: "CONSTITUENTS OF ZANTHOXYLUM RUGOSUM ST.-HIL & TUL" BIOCHEMICAL SYSTEMATICS AND ECOLOGY, PERGAMON PRESS, GB, vol. 3, no. 28, 2000, pages 275-277, XP008007208 ISSN: 0305-1978
- KANCHANAPOOM T ET AL: "CANTHIN-6-ONE AND BETA-CARBOLINE ALKALOIDS FROM EURYCOMA HARMANDIANA" PHYTOCHEMISTRY, PERGAMON PRESS, GB, vol. 56, no. 4, février 2001 (2001-02), pages 383-386, XP001086497 ISSN: 0031-9422 cité dans la demande
- HARRIS A ET AL: "CANTHIN-6-ONE ALKALOIDS FROM BRUCEA ANTIDYSENTERICA ROOT BARK" PLANTA MEDICA, THIEME, STUTTGART, DE, vol. 2, 1985, pages 151-153, XP008007205 ISSN: 0032-0943
- CHAN K L ET AL: "PLANTS AS SOURCES OF ANTIMALARIAL DRUGS. PART 3 EURYCOMA LONGIFOLIA" PLANTA MEDICA, THIEME, STUTTGART, DE, no. 2, 1986, pages 105-107, XP008007198 ISSN: 0032-0943
- KOIKE, KAZUO ET AL: "Synthesis and antitumor activity of canthin-5,6-dione derivatives" HETEROCYCLES (1999), 51(2), 315-323 , XP001180668
- FREIBURGHAUS F ET AL: "Evaluation of African medicinal plants for their in vitro trypanocidal activity" JOURNAL OF ETHNOPHARMACOLOGY, ELSEVIER SCIENTIFIC PUBLISHERS LTD, IE, vol. 55, 1996, pages 1-11, XP002212312 ISSN: 0378-8741
- FREIBURGHAUS F ET AL: "In vitro trypanocidal activity of some rare Tanzanian medicinal plants" ACTA TROPICA, ELSEVIER SCIENCE BV., AMSTERDAM, NL, vol. 67, no. 3, 1997, pages 181-185, XP002212313 ISSN: 0001-706X

## Description

L'invention a pour objet l'utilisation de la canthin-6-one, des extraits de plantes la contenant et de certains de ses dérivés pour la fabrication d'un médicament destiné au traitement des trypanosomiases, en particulier au traitement de la maladie de Chagas.

En Amérique Latine, environ 90 millions de personnes vivent dans des régions où la maladie de Chagas est endémique. Environ 18 à 20 millions de personnes sont déjà infectées par l'agent responsable de cette maladie : *Trypanozoma (Schizotrypanum) cruzi.*

Les traitements chimiothérapeutiques de cette maladie reposent à ce jour sur deux familles de molécules : les nitrofuranes, comme par exemple le nifurtimox, et les nitroimidazoles, comme par exemple le benznidazole. Ces composés peuvent avoir une efficacité sur la maladie de Chagas au début de l'infection, mais ils en ont peu ou pas du tout sur cette maladie lorsque *Trypanosoma cruzi* s'est installé dans l'organisme et que la maladie a pris un caractère chronique.

A ce stade, cette maladie est considérée à ce jour comme incurable.

Les traitements au nufurtimox et au benznidazole sont en outre confrontés à l'apparition de souches résistantes de *Trypanosoma cruzi,* ce qui diminue encore leur efficacité dans la phase première de la maladie de Chagas. Enfin, ces deux molécules ont des effets secondaires non négligeables tels que l'anorexie, les vomissements, la neuropathie périphérique et la dermopathie allergique.

Le besoin se faisait donc sentir d'un traitement de la maladie de Chagas qui soit efficace tant dans la première phase de la maladie, où *Trypanosoma cruzi* est présent essentiellement dans le sang, que dans la seconde phase de cette maladie, où l'on trouve *Tryponosoma cruzi* essentiellement dans les organes : coeur, système digestif.

La canthin-6-one est un composé connu qui a été isolé à partir de plantes telles que : *Ailanthus altissima* (Simaroubaceae) par Ohmoto et al., Chem. Pharm. Bull., 1976, 24, 1532-1536 ; *Brucea antidysenterica* (Simaroubaceae) par Fukamiya et al., Planta Med., 1987, 53, 140-143 ; *Eurycoma harmandiana* (Simaroubaceae) par Kachanapoom et al., Phytochemistry, 2001, 56, 383-386 ; *Peganum nigellastrum* (Zygophyllaceae) par Ma et al., Phytochemistry, 2000, 53, 1075-1078.

La canthin-6-one a été identifiée dans un extrait de *Zanthoxylum elephantiasis* (Rutaceae) par Mitscher et al., Lloydia, 1972, 35, 177-180.

Des activités thérapeutiques de la canthin-6-one ou d'extraits végétaux la contenant ont été rapportées dans les indications suivantes :
le traitement de la malaria par Kordono et al., J. Nat. Prod., 1991, 54(5), 1360-1367 ; comme agent antitumoral, par Fukamiya et al., Planta Med., 1987, 53(2), 140-143 ; comme agent antifongique par Mitscher et al., Lloydia, 1972, 35(2), 177-180.

*Zanthoxylum chiloperone,* d'où l'on a extrait la canthin-6-one pour la mise en oeuvre de l'invention, est connu pour son utilisation en médecine traditionnelle comme antiinflammatoire, comme antipyrrhétique, contre les rhumatismes et comme antiparasitaire général.

Le document WO 03/094990 décrit des oligo et polysaccharides et leur utilisation pour produire des surfaces hémocompatibles. Des molécules thérapeutiques peuvent être greffées sur cette surface. Parmi les agents anti-protozoaires qui peuvent être greffés sur cette surface sont cités la 1,11-diméthoxy canthin-6-one et la 1-hydroxy, 11-méthoxy canthin-6-one.

Le document O. Odebiyi et al., Planta Medica, 1979, 36, 204-207 décrit l'extraction de la canthin-6-one de la résine de *Fagara zanthoxyloides,* et son utlisation comme agent antimicrobien. Il est testé contre diverses infections bactériennes.

Le document M.E. Ferreira et al., Journal of ethnopharmacology, 80, 2002, 199-202 décrit deux composés, la canthin-6-one et la 5-méthoxy canthin-6-one et leur utilisation comme agent anti-protozoaire, pour le traitement des leishmanioses.

Le document E. Diehl et al., Biochemical Systematics and Ecology, 28, 2000, 275-277 décrit l'extraction de composants de *Zanthoxylum rugosum:* la canthin-6-one, la 5-méthoxy canthin-6-one.

Le document A. Harris et al., Planta Medica, 1985, vol. 2, p. 151-153 décrit l'extraction de la 1-hydroxy-11-méthoxy canthin-6-one et de la 11-hydroxy-1-méthoxy canthin-6-one à partir de *B. antidysenterica.* Il mentionne également l'existence de la 1-méthoxy canthin-6-one, de la 10-méthoxy canthin-6-one, DE la 5-méthoxycanthin-6-one et de la 10-hydroxy canthin-6-one.

Le document L. Chan et al., Planta Medica, 2, 1986, 105-107 décrit la 10-hydroxy canthin-6-one, son extraction *d'Eurycoma longifolia* et son utilisation comme agent antiplasmidien.

Le document Koike et al., Heterocycles, 51, n° 2, 1999, 315-323 décrit des composés dérivés de la canthin-5,6-dione.Un intermédiaire de synthèse, le chlorhydrate de N-méthyl 4-méthoxy 5-hydroxy canthin-6-one est divulgué par ce document.

Le document Freiburghaus F. et al., Journal of Ethnopharmacology, 55, 1996, 1-11 décrit l'utilisation d'extraits de plantes pour le traitement des trypanosomiases. Parmi ces plantes, l'extrait de tronc de *Zanthoxylum xanthoxyloïdes* présente une activité contre les trypanosomiases. Toutefois ce document ne mentionne pas la présence de la canthin-6-one ou de ses dérivés dans cet extrait de plante.

Le document F. Freiburghaus et al., Acta Tropica, 67, 1997, 181-185 décrit l'utilisation d'extraits de plantes pour le traitement d'une trypanosomiase : *Trypanosoma brucei.* Parmi les plantes utilisables est citée *Zanthoxylum chalibeum.* Aucun principe actif n'est mentionné.

Toutefois, rien dans l'art antérieur ne laissait supposer que la canthin-6-one était susceptible de constituer un traitement de la maladie de Chagas, tant dans sa phase première ou aiguë que dans sa phase chronique.

L'invention a donc pour objet l'utilisation de la canthin-6-one, d'extraits de plante la contenant et de certains de ses dérivés qui seront défmis ci-dessous pour la fabrication d'un médicament destiné au traitement des trypanosomiases, en particulier le traitement de la maladie de Chagas.

La canthin-6-one a été isolée à partir de l'écorce du tronc d'une rutacée identifiée comme *Zanthoxylum chiloperone* var. *angustifolium.*

Cette plante a été récoltée au Paraguay, près de Piribebuy dans le département de Cordillera. Un exemplaire de cette plante a été enregistré auprès de l'Herbarium de la Faculté de Chimie d'Asuncion au Paraguay sous le numéro AF917.

Par un procédé qui sera décrit ci-dessous ont été isolés plusieurs extraits de *Zanthoxylum chiloperone* var. *angustifolium.* On a également isolé la canthin-6-one elle-même à partir de cette plante. Toutefois on peut également mettre en oeuvre l'invention à partir de canthin-6-one isolée des autres plantes qui la contiennent et qui ont été énumérées plus haut. On peut également utiliser des extraits de *Ailanthus altissima,* de *Brucea antidysenterica,* d'*Eurycoma harmandiana,* de *Peganum Nigellastrum* ou de *Zanthoxylum elephantiasis* la contenant pour mettre en oeuvre l'invention.

Selon un mode préféré de réalisation de l'invention les extraits de *Zanthoxylum chiloperone* var. *angustifolium* et l'isolement de la canthin-6-one ont été réalisés suivant un procédé comportant une première étape qui consiste à broyer les écorces séchées du tronc de *Zanthoxylum chiloperone* var. *angustifolium* puis à les traiter par une solution alcaline aqueuse, comme par exemple par une solution aqueuse d'ammoniaque.

Le mélange obtenu est extrait par un solvant organique chloré comme par exemple du dichlorométhane.

La canthin-6-one peut ensuite être isolée et purifiée par des moyens bien connus de l'homme du métier tels que l'extraction, le lavage, la chromatographie, la précipitation, la recristallisation.

Le même procédé ou un procédé analogue peut être utilisé sur d'autres plantes contenant la canthin-6-one pour en obtenir des extraits comprenant la canthin-6-one ou pour isoler ce composé.

D'autres composés dérivés de la canthin-6-one peuvent être isolés des plantes citées ci-dessus par des méthodes analogues. On peut également préparer des dérivés de la canthin-6-one par des méthodes de synthèses bien connues de l'homme du métier en utilisant comme produit de départ la canthin-6-one ou tout autre composé approprié. En particulier, l'invention concerne les dérivés répondant à la formule (I) ci-dessous et leur utilisation pour la fabrication d'un médicament destiné au traitement d'une trypanosomiase :

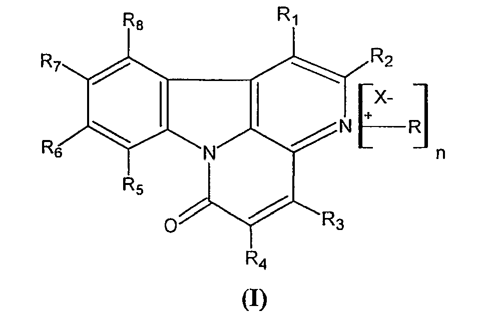

Dans la formule (I), R₁, R₂, R₃, R₄, R₅, R₆, R₇, R₈ représentent indépendamment l'un de l'autre :
- un atome d'hydrogène,
- un groupement alkyle en C₁-C₁₂ linéaire, ramifié ou cyclique, saturé ou insaturé,
- un atome d'halogène choisi parmi le chlore, le fluor, le brome et l'iode,
- un groupement halogénoalkyle en C₁-C₁₂ dont la chaîne alkyle peut être linéaire, ramifiée ou cyclique, saturée ou insaturée, et le ou les atomes d'halogène sont choisis parmi le fluor, le chlore, le brome et l'iode,
- une fonction hydroxyle,
- une fonction nitro -NO
- une fonction cyano -CN
- une fonction -SH
- une fonction acide carboxylique -COOH
- une fonction amide -CONH₂
- une fonction amine -NH₂
- une fonction alcoxy en C₁-C₁₂ dans laquelle le groupement alkyle peut être linéaire, ramifié ou cyclique, saturé ou insaturé,
- une fonction ester d'alkyle en C₁-C₁₂, dans laquelle le groupement alkyle peut être linéaire, ramifié ou cyclique, saturé ou insaturé
- une fonction alkyl amide secondaire ou tertiaire dans laquelle le ou les groupements alkyle en C₁-C₁₂ peuvent être linéaires, ramifiés ou cycliques, saturés ou insaturés,
- une fonction alkylamine secondaire ou tertiaire dans laquelle le ou les groupements alkyle en C₁-C₁₂ peuvent être linéaires, ramifiés ou cycliques, saturés ou insaturés,
- une fonction alkylthio en C₁-C₁₂, dans laquelle le groupement alkyle peut être linéaire, ramifié ou cyclique, saturé ou insaturé
- un groupement hétérocyclique en C₂-C₆ comportant 1 à 4 hétéroatomes choisis parmi le soufre, l'azote et l'oxygène,
- un groupement -SO₂-NR'R" ou un groupement -NR'-SO₂-R" dans lesquels R' et R " représentent indépendamment l'un de l'autre un groupement alkyle en C₁-C₁₂ linéaire, ramifié ou cyclique, saturé ou insaturé ;
   n représente 0 ou 1 ;
   R représente un groupement alkyle en C₁-C₁₂ linéaire, ramifié ou cyclique, saturé ou insaturé,
   X⁻ représente un anion qui peut être choisi parmi les anions minéraux ou organiques tels que par exemple l'ion Cl⁻, l'ion Br⁻, l'ion I⁻, l'ion S⁻, l'ion PO₃⁻, l'ion NO₃⁻, l'ion acétate, l'ion oxalate, l'ion tartarate, l'ion succinate, l'ion maléate, l'ion fumarate, l'ion gluconate, l'ion citrate, l'ion malate, l'ion ascorbate, l'ion benzoate.

La canthin-6-one correspond à la formule (I) dans laquelle :

R₁ = R₂ = R₃ = R₄ = R₅ = R₆ = R₇ = R₈ = H et n=0.

L'invention a donc pour objet un composé ainsi qu'un médicament comprenant ledit composé dans un support pharmaceutiquenent acceptable, ledit composé répondant à la formule (I) telle que définie ci-dessus, dans laquelle :
- l'un au moins de R₁, R₂, R₃, R₄, R₅, R₆, R₇ et R₈ est différent de H ou alors dans laquelle n=1, et
- lorsque n = 0, R₂ = R₃ = R₄ = R₅ = R₆ = R₇ = H et R₈ = OCH₃ alors R₁ est différent de -OH et de -OCH₃,
- lorsque n = 0, R₁ = R₂ = R₃ = R₅ = R₆ = R₇ = R₈ = H alors R₄ est différent de -OCH₃,
- lorsque n = 0, R₁ =R₂ = R₃ = R₄ = R₅ = R₇ = R₈ = H alors R₆ est différent de -OH et de -OCH₃,
- lorsque n = 0, R₁ = R₂ = R₃ = R₄ = R₅ = R₈ = H alors (R₆, R₇) est différent de (-OCH₃, -OCH₃),
- lorsque n = 0, R₂ = R₃ = R₄ = R₅ = R₆ = R₇ = R₈ = H alors R₁ est différent de -OCH₃,
- lorsque n = 0, R₁ = R₂ = R₃ = R₄ = R₅ = R₆ = R₈ = H alors R₇ est différent de -ohm
   et
   R₇ est différent de -OCH₃,
- lorsque n = 0, R₂ = R₃ = R₄ = R₅ = R₆ = R₇ = H et R₁ = -OCH₃ alors R₈ est différent de -OH,
   lorsque n = 1, X = Cl, R = CH₃, R₁ = R₂ = R₅ = R₆ = R₇ = R₈ = H et R₃ = -OCH₃ alors R₄ est différent de -OH.

Ledit composé sera appelé composé par la suite.

Préférentiellement, l'invention a pour objet l'un des composés A de formule (I) dans laquelle outre les conditions déjà mentionnées l'une ou plusieurs des conditions ci-dessous sont remplies :
- R₃ représente un groupement NH₂ ou un groupement alkylamine en C₁-C₁₂ ou un groupement alkylamide en C₁-C₁₂ ou un hétérocycle en C₂-C₆ comprenant au moins une fonction amine ;
- R₄ représente un groupement hydroxyle ou un groupement alcoxy en C₁-C₁₂ ;
- R₁ = R₂ = R₅ = R₆ = R₇ = R₈ = H.

Encore plus préférentiellement, l'invention a pour objet l'un des composés A de formule (I) dans laquelle outre les conditions déjà mentionnées l'une ou plusieurs des conditions ci-dessous sont remplies :
- R₃ représente un groupement NH₂ ou un groupement alkylamine en C₁-C₆ ou un groupement alkylamide en C₁-C₆ ou un hétérocycle en C₂-C₆ comprenant au moins une fonction amine ;
- R₄ représente un groupement hydroxyle ou un groupement alcoxy en C₁-C₆ ;
- R₁ = R₂ = R₅ = R₆ = R₇ = R₈ = H.

Encore plus préférentiellement, l'invention a pour objet l'un des composés A de formule (I) dans laquelle l'une ou plusieurs des conditions ci-dessous sont remplies :
- R₃ représente un groupement NH₂ ;
- R₄ représente un groupement OCH₃ ;
- R₁ = R₂ = R₅ = R₆ = R₇ = R₈ = H.

Selon une autre variante préférée de l'invention, le composé de l'invention est choisi parmi les composés de formule (I) dans laquelle R₁ = R₂ = R₃ = R₄ = R₅ = R₆ = R₇ = R₈ = H et n=1. Selon cette variante, avantageusement, R est un groupement alkyle en C₁-C₆.Encore plus avantageusement R est choisi parmi les groupements méthyle et éthyle.

De façon avantageuse, le composé de formule (I) est choisi parmi :
- la 4-amino canthin-6-one ;
- l'iodure de N-méthyl canthin-6-one ;

Les molécules de l'invention peuvent être obtenues en suivant l'une des voies de synthèse résumées dans les schémas ci-dessous. Les exemples de préparation donnés dans la partie expérimentale illustrent également des voies d'accès à ces composés. L'adaptation de ces voies de synthèse aux différents produits répondant à la formule (I) fait appel aux connaissances générales de l'homme du métier.

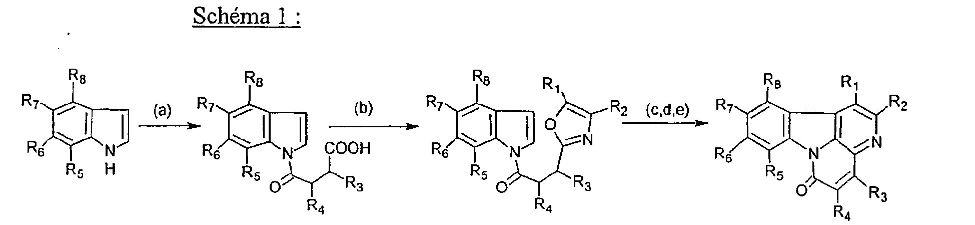

Légende : (a) anhydride succinique substitué ; (b) formation d'oxazoles substitués ; (c) réaction d'aza-Diels-Alder ; (d) déshydratation ; (e) oxydation de la liaison 4-5.

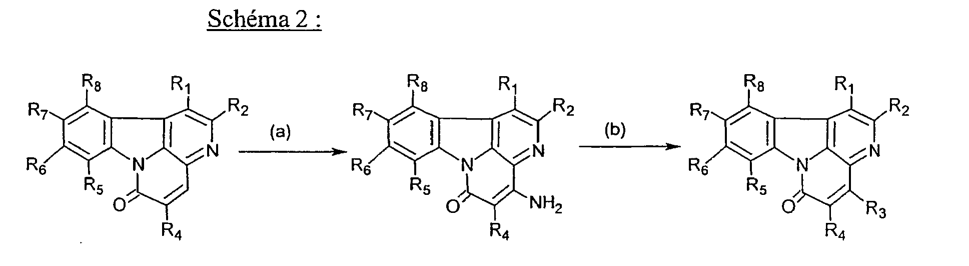

Légende : (a) voir exemple 2 ci-dessous ; (b) modifications de la fonction amine primaire.

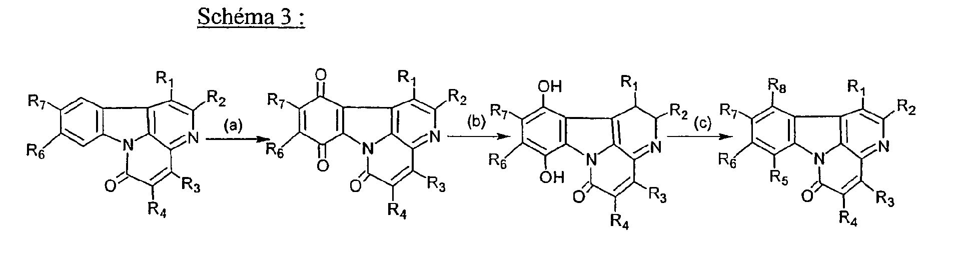

**Légende :** (a) oxydation en quinone ; (b) réduction ; (c) dérivatisations ou modifications des hydroxyles.

On connaît deux formes de trypanosomiases, l'une est causée par l'agent *Trypanosoma brucei* et est plus connue sous le nom de maladie du sommeil, l'autre est causée par l'agent *Trypanosoma cruzi* et est connue comme la maladie de Chagas. L'invention s'intéresse préférentiellement à la préparation d'un traitement efficace contre *Trypanosoma cruzi.*

La canthin-6-one a montré dans les tests d'activité qui sont exposés en détail ci-dessous une efficacité étonnante contre *Trypanosoma cruzi,* en particulier à des doses dix fois plus faibles que les doses auxquelles le benznidazole est efficace.

Selon l'invention, la canthin-6-one, des extraits de plantes la contenant, ou des dérivés de la canthin-6-one, tels que ceux répondant à la formule (I)définie ci-dessus, seront mis en oeuvre pour traiter des individus infectés par une typanosomiase, en particulier pour traiter des individus infectés par *Trypanosoma cruzi,* à une dose comprise entre 0,01 et 100 mg/kg/j de canthin-6-one ou d'un dérivé de formule (I), de préférence entre 0,1 et 50 mg/kg/j, encore plus préférentiellement entre 1 et 20 mg/kg/j.

Avantageusement, le traitement sera formulé sous forme de doses quotidiennes comprenant de 0,2 mg à 1 g de canthin-6-one ou d'un dérivé de formule (I), préférentiellement de 2 à 500 mg, encore plus préférentiellement de 5 à 200 mg.

La canthin-6-one, les extraits de plantes la contenant et ses dérivés de formule (I) peuvent être administrés par voie orale ou parentérale, associés à tout véhicule pharmaceutique approprié. Préférentiellement, la canthin-6-one les extraits de plantes la contenant et ses dérivés de formule (I) sont administrés par voie orale.

L'invention sera mieux comprise à l'aide des exemples suivants destinés à l'illustrer.

### EXEMPLES :

### Matériel et méthodes

Les spectres UV ont été obtenus sur un spectromètre Philips PU 8720. Les spectres IR ont été mesurés sur un spectromètre Perkin-Elmer 257 dans des pastilles de KBr. Les spectres NMR ¹H et ¹³C (CDCl₃) ont été obtenus sur un appareil Bruker AC-200 or AC-400 à une fréquence de respectivement 200 et 50 MHz ou de 400 et 100 MHz, respectively. Les EIMS et CIMS (méthane) ont été mesurés sur un spectromètre Nermag R10-10C. La CLHP semi-preparative a été faite à l'aide d'un détecteur Waters 590 relié à un enregistreur ABB SE 120, avec un système Millipore-Waters (Milford MA, USA) équipé d'une pompe 590, d'un injecteur SSV, et d'une colonne Prepak 1000 Millipore C₁₈.

### Exemple 1 : Isolement de la canthin-6-one et de la 5-méthoxy-canthin-6-one :

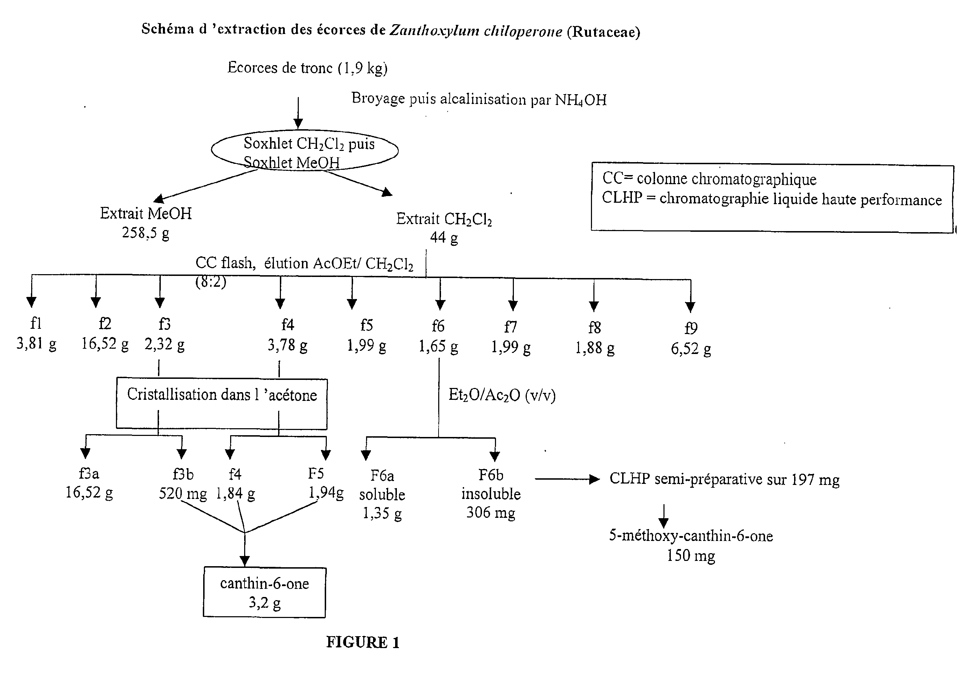

Le procédé d'extraction des écorces de *Zanthoxylum chiloperone* est représenté sur la figure 1 :

L'écorce séchée du tronc de *Zanthoxylum chiloperone* (1,9 kg) est traitée au dichlorométhane dans un appareil Soxhlet pour donner, après évaporation du solvant 44g d'extrait de plante. Cet extrait est redissout puis purifié par chromatographie flash sur colonne de silice en utilisant un mélange acétate d'éthyle/dichlorométhane (8 : 2) comme éluant. On récupère 9 fractions de 250 ml chacune, numérotées 1 à 9 dans l'ordre d'élution. Les fractions f_{3b} à f₅ sont combinées pour donner 3,2 g de la canthin-6-one après évaporation des solvants et cristallisation dans l'acétone.

La fraction f₆ est purifiée par CLHP préparative en utilisant comme solvant un mélange de méthanol et d'eau (7 : 3) pour donner 150 mg de 5-méthoxy-canthin-6-one après cristallisation dans l'acétone.

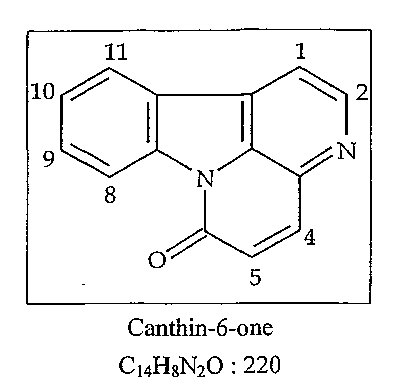

La canthin-6-one cristallise dans l'acétone sous forme d'aiguilles jaune pâle.

Le point de fusion (PF), déterminé sur banc de Köfler, est de 162°C.

**Spectre UV :** MeOHₘₐₓ nm (log ε) (dans le MeOH à 0,05g/l): 225 (1,70), 251 (1,35), 260 (1,40), 268 (1,40), 362 (1,33), 379 (1,29) ; (+ HCl 0,5N) : 225 (Non déterminable), 266 (1,49), 273 (1,49), 304 (1,56), 360 ; (+ NaOH 1N) : 225 (Non déterminable), 251 (1,54), 259 (1,55), 267 (1,50), 362 (1,33), 379 (1,29).

**Spectre IR** : 1665, 1630 cm⁻¹

**Spectre RMN ¹H :** 400 MHz (CDCl₃) _ ppm : 6,90 (d, 1H, J = 9,8 Hz, H₅) ; 7,50 (td, 1H, J= 8,5 ; 7,5 et 1 Hz, H₁₀) ; 7,70 (td, 1H, J= 8,2 ; 8,5 et 1Hz, H₉) ; 7,90 (d, 1H, *J=* 5 Hz, H₁) ; 8,00 (d, 1H, *J=* 9,8 Hz, H₄) ; 8,10 (dt, 1H, *J=* 7,5 et 1 Hz, H₁₁) ; 8,65 (dt, 1H, *J=* 8,2 et 1 Hz, H₈) ; 8,80 (d, 1H, *J=* 5 Hz, H₂).

**Spectre RMN ¹³C :** 50MHz (CDCl₃) _ ppm : 116,4 (C₁H), 117,2 (C₈H), 122,6 (C₁₁H), 124,3 (C₁₂), 125,7 (C₁₀H), 129,0 (C₅H), 130,1(C₁₃), 130,7 (C₉H), 131,9 (C₁₄), 136,2 (C₃ₐ), 139,3 (C₇ₐ), 139,6 (C₄H), 145,9 (C₂H), 159,0 (C₆).

**Spectre de masse :** [fragment ion] *m*/*z* (%) [M+Na]⁺243 (100%)

**Analyse élémentaire :** C : 76,42 ; H : 3,68 ; N:12,86 %.

### Exemple 2 : Procédé de synthèse de dérivés de la canthin-6-one

### ■ 4-amino canthin-6-one :

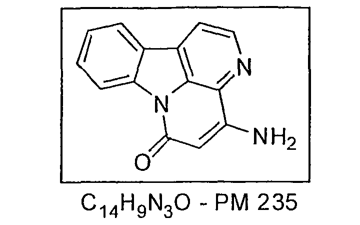

La canthin-6-one (100 mg - 0,45 mmol) est mise en suspension dans une solution saturée d'azoture de sodium (50 mL). Du diméthylformamide est ajouté jusqu'à l'obtention d'une solution limpide. Un excès de bromure de zinc est additionné (1 g) et le milieu porté à reflux jusqu'à consommation du produit de départ (réaction suivie par chromatographie sur couche mince, CH₂Cl₂/MeOH 9:1). Le milieu réactionnel refroidi est largement dilué par de l'eau puis extrait au dichlorométhane (4 fois). Les phases organiques réunies sont séchées (Na₂SO₄) puis concentrées sous pression réduite. La 4-amino canthin-6-one est purifiée par chromatographie « flash » sur colonne de silice (0,3 bar, élution : CH₂Cl₂/MeOH 95:5), 74 mg (70 %).

On obtient un solide poudreux jaune : **spectre RMN ¹H** (400 MHz, CDCl₃) : δ ppm, 4,9 (s, 2 H) ; 7,0 (s, 1 H) ; 7,5 (t, *J*= 7,6 Hz, 1 H) ; 7,7 (m, 2 H) ; 8,05 (d, *J* = 7,6 Hz, 1 H) ; 8,65 (d, *J* = 8,1 Hz, 1 H) ; 8,7 (d, *J* = 5,1 Hz, 1 H) ; **spectre RMN ¹³C** (100 MHz, CDCl₃) δ ppm, 106,8 ; 112,0 ; 117,0 ; 122,6 ; 125,7 ; 125,8 ; 126,5 ; 129,1 ; 130,1 ; 138,8 ; 139,1 ; 142,4 ; 145,9 ; 156,2 ; **spectre Infra-rouge** (ν, cm⁻¹) : 3254, 1673, 1612, 1580, 1556, 1443, 1333, 1313 ; **spectre de masse** (électrospray, *m*/*z*) : 236 [M+H⁺]; **PF** (CH₂Cl₂) : 199-200 °C ; ***R**_{f}*= 0,6 (CH₂Cl₂/MeOH 9:1).

### ■ Iodure de N-méthyl canthin-6-one

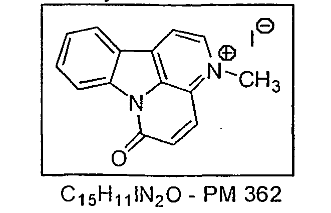

La canthin-6-one (100 mg - 0,45 mmol) est mise en solution dans de l'iodure de méthyle (1 mL). La solution est agitée à température ambiante jusqu'à consommation du produit de départ (réaction suivie par chromatographie sur couche mince, CH₂Cl₂/MeOH 9:1). Le précipité est recueilli par filtration et lavé au dichlorométhane (150 mg - 90 %).

On obtient une poudre orange, **spectre RMN ¹H** (400 MHz, DMSO-*d₆*) : δ ppm, 4,6 (s, 3 H) ; 7,4 (d, *J* = 10,0 Hz, 1 H) ; 7,7 (t, *J* = 7,7 Hz, 1 H) ; 8.0 (t, *J* = 7,8 Hz, 1 H) ; 8,6 (m, 3 H) ; 8,9 (d, *J* = 6,3 Hz, 1 H) ; 9,1 (d, *J* = 6,3 Hz, 1 H) ; **spectre RMN ¹³C** (100 MHz, CDCl₃) δ ppm, 44,3 ; 116,8 ; 119,1 ; 122,5 ; 125,7 ; 127,4 ; 127,5 ; 130,2 ; 133,3 ; 133,7 ; 134,7 ; 136,1 ; 141,4 ; 142,7 ; 158,0 ; **spectre Infra-rouge** (ν, cm⁻¹) : 1684, 1655, 1340, 1257, 1142 ; **spectre de masse** (électrospray, *m*/*z*) : 235 [M⁺] ; PF (CH₂Cl₂) : 240 °C.

### Exemple 3 : Méthodologie des essais in vivo sur Trypanosoma cruzi en phase aigüe :

Animaux et parasites : Les souris de type Balb/c sont élevées dans l'animalerie de l'Institut d'Investigations des Sciences Médicales (IICS, Asuncion, Paraguay) et sont âgées de 6 à 8 semaines lors des protocoles expérimentaux.

Pour ces essais, la souche CL (clône Brener) de *T. cruzi* est employée sous la forme circulante du parasite (trypomastigotes). Les animaux sont infectés par voie intrapéritonéale avec 5000 parasites, cette souche produit son pic de parasites 21 à 25 jours après l'infection. Chaque semaine le nombre de parasites est contrôlé par prélèvement sanguin au niveau de la queue de la souris.

Infection et traitement : Les traitements avec le benznidazole, le médicament de référence et la canthin-6-one débutent 11 jours après l'infection parasitaire à raison de 50 mg/kg ou 200 mM/kg pour le benznidazole et à la concentration de 5 mg/kg ou 20 mM/kg pour la canthin-6-one. La durée des traitements est fixée à deux semaines et la voie d'administration choisie est la voie orale pour le benznidazole et la canthin-6-one, par ailleurs un groupe de souris est traité avec la canthin-6-one par voie sous cutanée. Les souris non traitées et infectées reçoivent 100 µl d'une solution saline phosphatée tamponnée.

Critères d'évaluation de l'efficacité des traitements :
- comptage hebdomadaire du nombre de parasites circulant dans le sang périphérique pendant la durée de l'expérimentation, soit 10 semaines.
- observation de la mortalité,
- deux évaluations sérologiques, 40 jours post-infection soit 15 jours après cessation du traitement et 68 jours post-infection soit 45 jours post-traitement. L'évaluation sérologique est effectuée à l'aide d'un test ELISA (enzyme linked immunoassay) kit Chagas, IISC, Asuncion. Les densités optiques sont mesurées avec un lecteur de plaques ELISA (Titerek, Unistan, I).

Etudes statistiques : La moyenne et les déviations standard de chaque groupe sont calculées, les différences entre les groupes sont déterminées par le test de Student et le test non-paramétrique d'analyse de variance de Kruskal-Wallis. Les comparaisons s'effectuent entre le groupe non traité et les groupes traités, P<0,05.

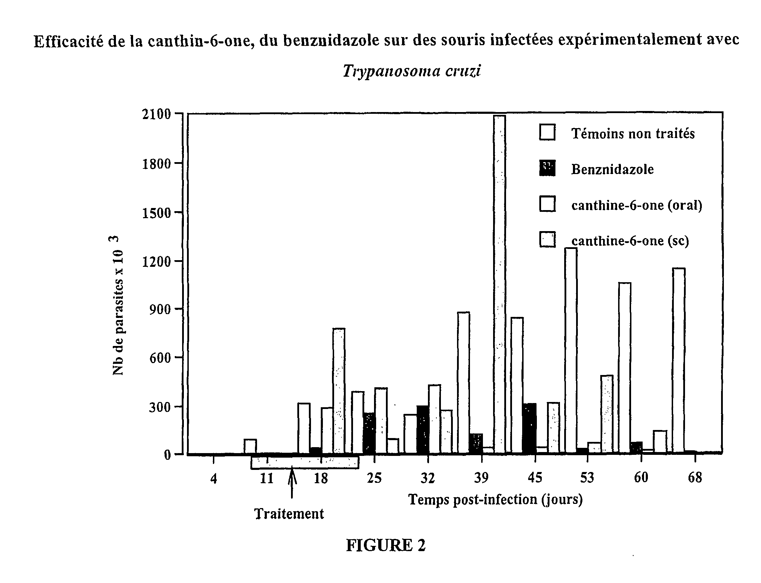

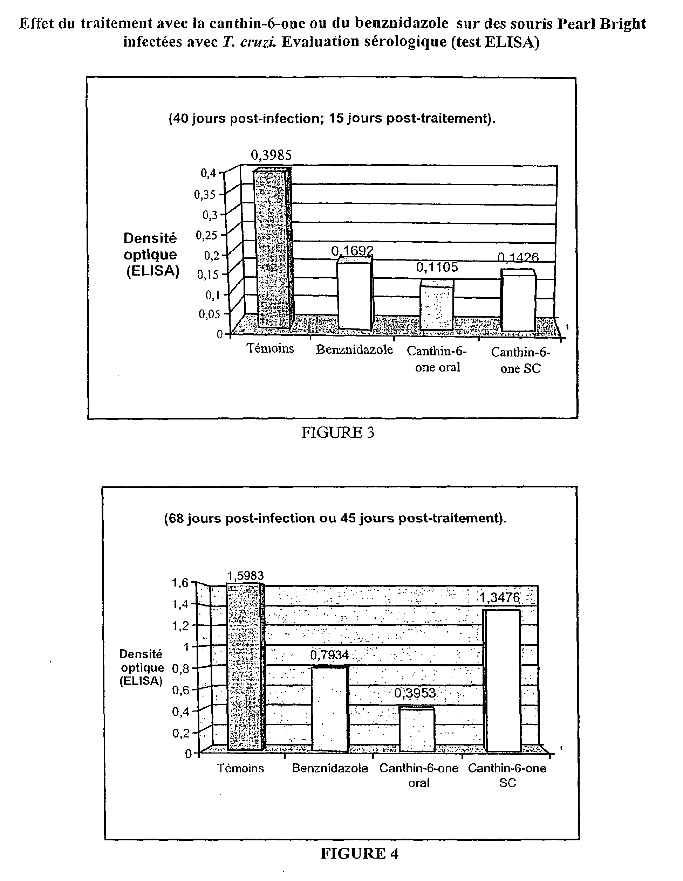

Les résultats sont exposés dans les tableaux I et II et sur les figures 2, 3 et 4.

**TABLEAU I**

| **Efficacité de la canthin-6-one, du benznidazole sur des souris infectées expérimentalement avec *Trypanosoma cruzi*** | | | | |
|---|---|---|---|---|
| **Evaluation parasitologique (Nombre de parasites ± Déviation standard)** | | | | |
| Jours post-infection | Témoins non traités (n = 8) | Benznidazole (n = 8) | Canthin-6-one Oral (n = 7) | Canthin-6-one Sous cutanée (n = 8) |
| 4 | 0 | 0 | 0 | 0 |
| 11* | 90.9 ± 257 | 0 | 0 | 0 |
| 18* | 313.6 ± 468.7 | 34.9 ± 98.6 | 286 ± 515.9 | 766.3 ± 719.2 |
| 25* | 387.3 ± 671.1 | 250.1 ± 503.5 | 402 ± 837.7 | 88.4 ± 142.9 |
| 32 | 242.1 ± 553.2 | 296.8 ± 625.5 | 426.2 ± 664.5 | 267.5 ± 546.5 |
| 39 | 870.5 ± 1902.1 | 118.3 ± 192.9 | 36.6 ± 58.4 | 2077.1 ± 2214.2 |
| 45 | 835.8 ± 1022.7 | 300.8 ± 431.6 | 34.4 ± 76.9 | 314.1 ± 499.3 |
| | | | P=0.05 | |
| 53 | 1273.3 ± 1647.8 | 23.3 ± 65.8 | 58.4 ± 80.6 | 473.4 ± 921.9 |
| | | P=0.01 | P=0.05 | |
| 60 | 1050.1 ± 2605.5 | 65.3 ± 93.2 | 16 ± 35.8 | 129.9 ± 194.4 |
| | | | P<0.05 | |
| 68 | 1144.1 ± 1641.9 | 9.4 ± 26.5 | 0 | 34.9 ± 98.6 |
| | | P=0.03 | P=0.02 | P=0.03 |

| | | | | |
|---|---|---|---|---|
| * Période de traitement (deux semaines) n = nombre de souris | | | | |

**TABLEAU II :**

| Effet du traitement avec la canthin-6-one ou du benznidazole sur des souris Balb/c infectées avec *T. cruzi.* | | | | | | |
|---|---|---|---|---|---|---|
| Evaluation sérologique (test ELISA) | | | | | | |
| Traitement | Nb Souris | Voie adm. | 1^{a} sérologíe ® | Sérologie négative/ survivant | 2^{a} sérologie ∇ | Sérologie négative/ survivant |
| Témoins non traités (PBS) | 8 | Oral | 0.3985 ± 0.092 | 0 / 8 (0%) | 0,1598 ± 0,382.3 | 0 / 8 (0%) |
| Benznidazole (médicament de référence) (50 mg) | 8 | Oral | 0.1692 ± | 6/8 (75%) | 0,7934 ± 0,8607 | 3 / 8 (37.5% |
| | | | 0.1179 | | P < 0.05 | |
| | | | P < 0.001 | | | |
| Canthin-6-one (5 mg) | 7 | Oral | 0.1105 | ± 7 / 7 (100%) | 0,3953 ± 0,7531 | 3 / 7 (42.9%) |
| | | | 0.0387 | | P < 0.05 | |
| | | | P < 0.001 | | | |
| Canthin-6-one ( 5 mg) | 8 | SC | 0.2151± | 4 / 7 (57.1%) | 0,1347 ± 0,6327 | 2/6 (33.3%) |
| | | | 0.1447 | | P < 0.001 | |
| | | | P < 0.05 | | | |

| | | | | | | |
|---|---|---|---|---|---|---|
| Sérologie: ELISA anti-***T. cruzi***. ® 40 jours post-infection; 15 jours post-traitement ∇ 68 jours post-infection; 45 jours post-traitement. Valeur de *P* versus témoins non traités | | | | | | |

Comme on peut le constater sur la figure 2, la canthin-6-one administrée par voie orale, à une dose de 5 mg/kg/j montre à partir du 39^{ème} jour après l'infestation, et 15 jours après la fin du traitement, une activité bien supérieure au benznidazole employé à la dose de 50 mg/kg/j. Elle permet une éradication totale de *Trypanosoma cruzi* de l'organisme infecté, ce qui ne permet pas d'obtenir le benznidazole. Ces résultats sont confirmés par la mesure de la densité optique (ELISA) à 15 et 48 jours après la fin du traitement comme cela est illustré sur les figures 3 et 4.

### Exemple 4 : Méthodologie des essais in vivo sur Trypanosoma cruzi en phase chronique

### Animaux et parasites :

Les souris de type Balb/c sont élevées dans l'animalerie de l'Institut d'Investigations des Sciences de la Santé (IICS, Asuncion, Paraguay et sont âgées de 6 à 8 semaines lors des protocoles expérimentaux. Pour ce protocole expérimental, la souche CL (clône Brener) de *T. cruzi* est utilisée sous la forme circulante (trypomastigotes), la souche est maintenue en culture de routine sur modèle animal par passage tous les 14 jours. Les animaux sont infectés par voie intrapéritonéale avec 1000 parasites. Dans ces conditions expérimentales, il se produit un lent développement des parasites, cette souche produit son pic de parasites 21 à 28 jours après l'infection. La majorité des souris survivent (70-80 %) avec une légère détérioration de leur état général physique et avec une parasitémie absente ou sub-patente. Chaque semaine le nombre de parasites est contrôlé par prélèvement au niveau de la queue de la souris.

### Infection et traitements :

Pour cette expérimentation de longue durée, les traitements démarrent 120 jours après l'infection parasitaire quand la parasitémie est sub-patente chez toutes les souris. Les souris sont ensuite divisées en groupes au hasard. Les traitements avec le benznidazole, le médicament de référence, sont administrés à la concentration de 50 mg/kg ou 200 mM/kg par jour pendant 20 jours par voie orale. La canthin-6-one est administrée soit par voie orale, soit par voie sous-cutanée à la concentration de 5 mg/kg ou 20 mM/kg par jour pendant 20 jours. Un extrait total dichlorométhanique d'écorces de tronc de *Zanthoxylum chiloperone* var. *angustofium* est administré soit par voie orale, soit par voie sous-cutanée à la concentration de 50 mg/kg par jour pendant 20 jours. Pour l'administration, les principes actifs sont dissous dans 50 µl d'une solution saline phosphatée tamponnée (PBS). Les souris non traitées et infectées reçoivent 50 µl de PBS.

### Critères d'évaluation de l'efficacité des traitements :

- comptage hebdomadaire du nombre de parasites circulant dans le sang périphérique pendant la durée de l'expérimentation, soit 30 semaines.
- Observation de la mortalité
- Trois évaluations sérologies, 45 jours avant le début des traitements, 10 jours après la cessation du traitement et 75 jours post-traitement. L'évaluation sérologique est effectuée à l'aide d'un test ELISA (enzyme linked immuno assay) kit Chagas, IICS, Asuncion. Les densités optiques sont mesurées avec un lecteur de plaques ELISA (Titerek Unistan I).

### Etudes statistiques :

La moyenne et les déviations standard de chaque groupe sont calculées, les différences entre les groupes sont déterminées par le test de Student et le test non-paramétrique d'analyse de variance de Kruskal-Wallis. Les comparaisons s'effectuent entre le groupe non traité et les groupes traités, P<0,05. Les résultats sont présentés dans les tableaux III et IV.

**Tableau III**

| Cures parasitologiques chez des souris infectées chroniquement par *T. cruzi* et traitées pendant 20 jours avec le benznidazole (*n*=5), la canthin-6-one (*n*=8) et un extrait total de *Zanthoxylum chiloperone* var.*angustifolium* (*n*=7) | | | | |
|---|---|---|---|---|
| Traitement* | Parasitémie négative/nombre de souris survivantes (nombre de jours post-traitement) | | | |
| Souris témoins non traitées | 0 | 10j | 40j | 60j |
| | 5/5 | 2/4 | 1/1 | 1/1 |
| Benznidazole (50 mg/kg/j) voie orale | 5/5 | 2/5 | 5/5 | 5/5 |
| Canthin-6-one (5 mg/kg) voie orale | 7/8 | 7/8 | 8/8 | 8/8 |
| Canthin-6-one (5 mg/kg/j) voie sous-cutanée | 6/8 | 7/8 | 6/8 | 6/8 |
| Extrait total d'écorces de *Z. chiloperone* (50 mg/kg/j) voie orale | 7/7 | 7/7 | 7/7 | 7/7 |
| Extrait total d'écorces de *Z. chiloperone* (50 mg/kg/j) voie sous-cutanée | 4/6 | 4/6 | 5/5 | 3/5 |

| | | | | |
|---|---|---|---|---|
| * traitements 108 jours après l'infection parasitaire | | | | |

**Tableau IV**

| Effet du traitement avec la canthin-6-one, ou un extrait total de *Zanthoxylum chilopenone* var. *angustifolium* ou du benznidazole sur des souris Balb/c infectées chroniquement avec *T. cruzi.* | | | |
|---|---|---|---|
| Traitement | ELISA (densité optique ± déviation standard) Nombre de jours post-traitement | | |
| | 43 jours avant-traitement | 10 j | 75 j |
| Souris témoins non traitées | 1,805 ± 0,075 | 1,913 ± 0,115 | 1,793* |
| Benznidazole (50 mg/kg/j) voie orale | 2,072 ± 0,220 | 1,712 ± 0,473 | 1,979 ± 0,350 |
| Canthin-6-one (5 mg/kg) voie orale | 1,878 ± 0,348 | 1,621 ± 0,547 | 1,799 ± 0,333 |
| Canthin-6-one (5 mg/kg/j) voie sous-cutanée | 1,916 ± 0,368 | 1,850 ± 0,405 | 1,870 ± 0,268 |
| Extrait total d'écorces de *Z*. *chiloperone* (50 mg/kg/j) voie orale | 1,932 ± 0,228 | 1,890 ± 0,288 | 1,961 ± 0,172 |
| Extrait total d'écorces de *Z. chiloperone* (50 mg/kg/j) voie sous-cutanée | 1,718 ± 0,264 | 1,703 ± 0,470 | 1,815 ± 0,374 |

| | | | |
|---|---|---|---|
| * une seule souris vivante en fin d'expérimentation | | | |

Comme on peut le constater dans le tableau III, la canthin-6-one administrée par voie orale, à une dose de 5 mg/kg/j pendant 20 jours à partir du 108^{ème} jour après l'infection parasitaire, et 79 jours après la fin du traitement, a montré une activité supérieure au benznidazole employé à la dose de 50 mg/kg/j. Elle induit une éradication totale de *Trypanosoma cruzi* de l'organisme infecté et protège les souris de la mort. Ces résultats sont confirmés en sérologie par le test ELISA à 10 et 75 jours après la fin du traitement comme cela est illustré par les données du tableau IV.

## Revendications

1. Utilisation pour la fabrication d'un médicament destiné au traitement d'une trypanosomiase d'un composé répondant à la formule (I) : 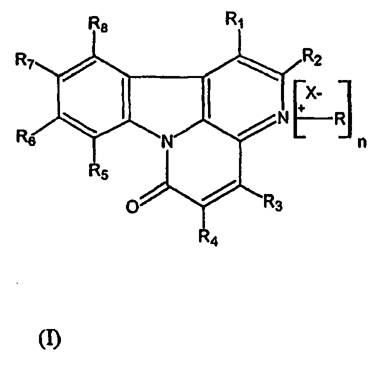 dans laquelle R₁, R₂, R₃, R₄, R₅, R₆, R₇, R₈ représentent indépendamment l'un de l'autre :
• un atome d'hydrogène,
• un groupement alkyle en C₁-C₁₂ linéaire, ramifié ou cyclique, saturé ou insaturé,
• un atome d'halogène choisi parmi le chlore, le fluor, le brome et l'iode,
• un groupement halogénoalkyle en C₁-C₁₂ dont la chaîne alkyle peut être linéaire, ramifiée ou cyclique, saturée ou insaturée, et le ou les atomes d'halogène sont choisis parmi le fluor, le chlore, le brome et l'iode,
• une fonction hydroxyle,
○ une fonction nitro NO
○ une fonction cyano -CN
○ une fonction OH
○ une fonction acide carboxylique -COOH
○ une fonction amide -CONH₂
• une fonction amine NH₂
• une fonction alcoxy en C₁-C₁₂ dans laquelle le groupement alkyle peut être linéaire, ramifié ou cyclique, saturé ou insaturé,
• une fonction ester d'alkyle en C₁-C₁₂, dans laquelle le groupement alkyle peut être linéaire, ramifié ou cyclique, saturé ou insaturé
• une fonction alkyl amide secondaire ou tertiaire dans laquelle le ou les groupements alkyle en C₁-C₁₂ peuvent être linéaires, ramifiés ou cycliques, saturés ou insaturés,
• une fonction alkylamine secondaire ou tertiaire dans laquelle le ou les groupements alkyle en C₁-C₁₂ peuvent être linéaires, ramifiés ou cycliques, saturés ou insaturés,
• une fonction alkylthio en C₁-C₁₂, dans laquelle le groupement alkyle peut être linéaire, ramifié ou cyclique, saturé ou insaturé,
• un groupement hétérocyclique en C₂-C₆ comportant 1 à 4 hétéroatomes choisis parmi le soufre, l'azote et l'oxygène,
• un groupement -SO₂-NR'R" ou un groupement NR'-SO₂-R" dans lesquels R' et R " représentent indépendamment l'un de l'autre un groupement alkyle en C₁-C₁₂ linéaire, ramifié ou cyclique, saturé ou insaturé ;
n représente 0 ou 1 ;
R représente un groupement alkyle en C₁-C₁₂ linéaire, ramifié ou cyclique, saturé ou insaturé,
X⁻ représente un anion qui peut être choisi parmi les anions minéraux ou organiques.

2. Utilisation selon la revendication 1, **caractérisée en ce que** le composé de formule (I) est la canthin-6-one.

3. Utilisation de la canthin-6-one, pour la fabrication d'un médicament destiné au traitement d'une trypanosomiase selon la revendication 2, **caractérisée en ce que** la canthin-6-one est présente sous forme d'un extrait de plante.

4. Utilisation selon la revendication 3, **caractérisée en ce que** la canthin-6-one est présente sous forme d'un extrait d'une plante choisie parmi : *Ailanthus altissima, Brucea antidysenterica, Eurycoma harmandiana, Peganum nigellastrum, Zanthoxylum elephantiasis* et *Zanthoxylum chiloperone.*

5. Utilisation selon la revendication 4, **caractérisée en ce que** la canthin-6-one est présente sous forme d'un extrait de *Zanthoxylum chiloperone* var. *angustifolium.*

6. Utilisation selon l'une quelconque des revendications 1 à 5, pour la fabrication d'un médicament destiné au traitement d'une trypanosomiase dans sa phase chronique et sa phase aiguë.

7. Utilisation selon l'une quelconque des revendications 1 à 5, pour la fabrication d'un médicament destiné au traitement de la maladie de Chagas.

8. Utilisation selon l'une quelconque des revendications 1 à 6 précédentes, **caractérisée en ce qu'**elle est destinée au traitement d'une trypanosomiase causée par l'agent *Trypanosoma brucei.*

9. Utilisation selon l'une quelconque des revendications 1 à 7 précédentes, **caractérisée en ce qu'**elle est destinée au traitement d'une trypanosomiase causée par l'agent *Trypanosoma cruzi*

10. Utilisation selon la revendication 5, **caractérisée en ce que** l'extrait de plante contenant la canthin-6-one est obtenu par un procédé comportant une première étape qui consiste à broyer les écorces séchées du tronc de *Zanthoxylum chiloperone* var. *angustifolium* puis à les traiter par une solution alcaline aqueuse.

11. Utilisation selon la revendication 10, **caractérisée en ce que** l'extrait de plante contenant la canthin-6-one est obtenue par un procédé comportant une seconde étape consistant en une extraction par un solvant organique chloré.

12. Utilisation selon l'une quelconque des revendications 1 à 11 précédentes, **caractérisée en ce que** le médicament est destiné à être administré à une dose comprise entre 0,01 et 100 mg/kg/j de composé de formule (I), de préférence entre 0,1 et 50 mg/kg/j, encore plus préférentiellement entre 1 et 20 mg/kg/j.

13. Utilisation selon l'une quelconque des revendications précédentes, **caractérisée en ce que** le médicament est destiné à être administré par voie orale.

14. Composé répondant à la formule (I) : 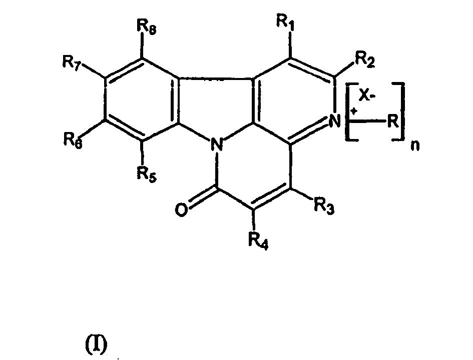 dans laquelle R₁, R₂, R₃, R₄, R₅, R₆, R₇, R₈ représentent indépendamment l'un de l'autre :
○ un atome d'hydrogène,
○ un groupement alkyle en C₁-C₁₂ linéaire, ramifié ou cyclique, saturé ou insaturé,
• un atome d'halogène choisi parmi le chlore, le fluor, le brome et l'iode,
• un groupement halogénoalkyle en C₁-C₁₂ dont la chaîne alkyle peut être linéaire, ramifiée ou cyclique, saturée ou insaturée, et le ou les atomes d'halogène sont choisis parmi le fluor, le chlore, le brome et l'iode,
• une fonction hydroxyle,
• une fonction nitro -NO
• une fonction cyano -CN
• une fonction -SH
• une fonction acide carboxylique -COOH
• une fonction amide -CONH₂
• une fonction amine NH₂
• une fonction alcoxy en C₁-C₁₂ dans laquelle le groupement alkyle peut être linéaire, ramifié ou cyclique, saturé ou insaturé,
• une fonction ester d'alkyle en C₁-C₁₂, dans laquelle le groupement alkyle peut être linéaire, ramifié ou cyclique, saturé ou insaturé
• une fonction alkyl amide secondaire ou tertiaire dans laquelle le ou les groupements alkyle en C₁-C₁₂ peuvent être linéaires, ramifiés ou cycliques, saturés ou insaturés,
• une fonction alkylamine secondaire ou tertiaire dans laquelle le ou les groupements alkyle en C₁-C₁₂ peuvent être linéaires, ramifiés ou cycliques, saturés ou insaturés,
• une fonction alkylthio en C₁-C₁₂, dans laquelle le groupement alkyle peut être linéaire, ramifié ou cyclique, saturé ou insaturé,
• un groupement hétérocyclique en C₂-C₆ comportant 1 à 4 hétéroatomes choisis parmi le soufre, l'azote et l'oxygène
• un groupement -SO₂-NR'R" ou un groupement -NR'-SO₂-R" dans lesquels R' et R" représentent indépendamment l'un de l'autre un groupement alkyle en C₁-C₁₂ linéaire, ramifié ou cyclique, saturé ou insaturé ;
n représente 0 ou 1 ;
R représente un groupement alkyle en C₁-C₁₂ linéaire, ramifié ou cyclique, saturé ou insaturé,
X⁻ représente un anion qui peut être choisi parmi les anions minéraux ou organiques, étant entendu que :
○ l'un au moins de R₁, R₂, R₃, R₄, R₅, R₆, R₇ et R₈ est différent de H, ou alors n=1, et
○ lorsque n = 0, R₂ = R₃ = R₄ = R₅ = R₆ = R₇ = H et R₈ = OCH₃ alors R₁ est différent de -OH et de -OCH₃,
• lorsque n = 0, R₁ = R₂ = R₃ = R₅ = R₆ = R₇ = R₈ = H alors R₄ est différent de -OCH₃,
• lorsque n = 0, R₁ =R₂ = R₃ = R₄ = R₅ = R₇ = R₈ = H alors R₆ est différent de -OH et de -OCH₃,
• lorsque n = 0, R₁ = R₂ = R₃ = R₄ = R₅ = R₈ = H alors (R₆, R₇) est différent de (-OCH₃, -OCH₃),
• lorsque n = 0, R₂ = R₃ = R₄ = R₅ = R₆ = R₇ = R₈ = H alors R₁ est différent de -OCH₃,
• lorsque n = 0, R₁ = R₂ = R₃ = R₄ = R₅ = R₆ = R₈ = H alors R₇ est différent de -OH
et
R₇ est différent de -OCH₃,
• lorsque n = 0, R₂ = R₃ = R₄ = R₅ = R₆ = R₇ = H et R₁ = -OCH₃ alors R₈ est différent de -OH,
• lorsque n = 1, X = Cl, R = CH₃, R₁ = R₂ = R₅ = R₆ = R₇ = R₈ = H et R₃ = -OCH₃ alors R₄ est différent de -OH.

15. Composé selon la revendication 14, **caractérisé en ce que** X- est choisi parmi : l'ion Cl⁻, l'ion Br⁻, l'ion I⁻, l'ion S⁻, l'ion PO₃⁻; l'ion NO₃⁻, l'ion acétate, l'ion oxalate, l'ion tartarate, l'ion succinate, l'ion maléate, l'ion fumarate, l'ion gluconate, l'ion citrate, l'ion malate, l'ion ascorbate, l'ion benzoate.

16. Composé selon la revendication 14 ou la revendication 15, **caractérisé en ce que** l'une ou plusieurs des conditions ci-dessous sont remplies :
- R₃ représente un groupement NH₂ ou un groupement alkylamine en C₁-C₁₂ ou un groupement alkylamide en C₁-C₁₂ ou un hétérocycle en C₂-C₆ comprenant au moins une fonction amine ;
- R₄ représente un groupement hydroxyle ou un groupement alcoxy en C₁-C₁₂ ;
- R₁ = R₂ = R₅ = R₆ = R₇ = R₈ = H.

17. Composé selon l'une quelconque des revendications 14 à 16, **caractérisé en ce que** l'une ou plusieurs des conditions ci-dessous sont remplies :
- R₃ représente un groupement NH₂ ou un groupement alkylamine en C₁-C₆ ou un groupement alkylamide en C₁-C₆ ou un hétérocycle en C₂-C₆ comprenant au moins une fonction amine ;
- R₄ représente un groupement hydroxyle ou un groupement alcoxy en C₁-C₆ ;
- R₁ = R₂ = R₅ = R₆ = R₇ = R₈ = H.

18. Composé selon l'une quelconque des revendications 14 à 17, **caractérisé en ce que** l'une ou plusieurs des conditions ci-dessous sont remplies :
- R₃ représente un groupement NH₂ ;
- R₄ représente un groupement OCH₃ ;
- R₁ = R₂ = R₅ = R₆ = R₇ = R₈ = H.

19. Composé selon l'une quelconque des revendications 14 à 18, **caractérisé en ce que** R₁ = R₂ = R₃ = R₄ = R₅ = R₆ = R₇ = R₈ = H et n=1, R est un groupement alkyle en C₁-C₆.

20. Composé selon l'une quelconque des revendications 14 à 18, **caractérisé en ce qu'**il est choisi parmi :
- la 4-amino canthin-6-one ;
- l'iodure de N-méthyl canthin-6-one.

21. Médicament, **caractérisé en ce qu'**il comprend un composé selon l'une quelconque des revendications 14 à 20 dans un support pharmaceutiquement acceptable.

## Claims

1. Use, for the preparation of a medicine intended for the treatment of trypanosomiasis, of a compound in accordance with formula (I): 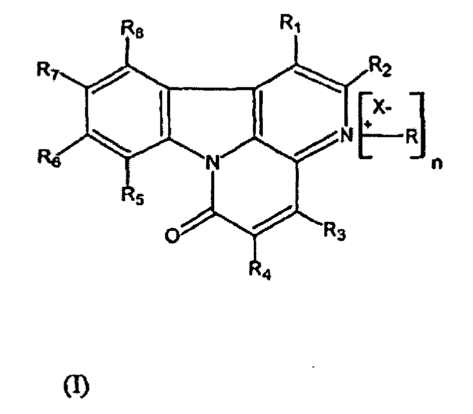 in which R₁, R₂, R₃, R₄, R₅, R₆, R₇, R₈ represent independently of one another:
• a hydrogen atom,
• a C₁-C₁₂ alkyl group that is linear, branched or cyclic, saturated or unsaturated,
• a halogen atom selected from chlorine, fluorine, bromine and iodine,
• a C₁-C₁₂ alkyl halide group, of which the alkyl chain can be linear, branched or cyclic, saturated or unsaturated, and the halogen atom or atoms is or are selected from fluorine, chlorine, bromine and iodine,
• a hydroxyl function
○ an -NO nitro function
○ a -CN cyano function
○ an -SH function
○ a -COOH carboxylic acid function
○ a -CONH₂ amide function
• an -NH₂ amine function
• a C₁-C₁₂ alkoxy function, in which the alkyl group can be linear, branched or cyclic, saturated or unsaturated,
• a C₁-C₁₂ alkyl ester function, in which the alkyl group can be linear, branched or cyclic, saturated or unsaturated,
• a secondary or tertiary alkylamide function, in which the C₁-C₁₂ alkyl group or groups can be linear, branched or cyclic, saturated or unsaturated,
• a secondary or tertiary alkylamine function, in which the C₁-C₁₂ alkyl group or groups can be linear, branched or cyclic, saturated or unsaturated,
• a C₁-C₁₂ alkylthio function, in which the alkyl group can be linear, branched or cyclic, saturated or unsaturated,
• a C₂-C₆ heterocyclic group comprising 1 to 4 heteroatoms selected from sulphur, nitrogen and oxygen,
• an -SO₂-NR'R" group or an -NR'-SO₂R" group, in which R' and R" represent independently of one another a C₁-C₁₂ alkyl group that is linear, branched or cyclic, saturated or unsaturated;
n represents 0 or 1;
R represents a C₁-C₁₂ alkyl group that is linear, branched or cyclic, saturated or unsaturated,
X represents an anion that can be selected from inorganic or organic anions.

2. Use according to claim 1, **characterised in that** the compound of formula (I) is canthin-6-one.

3. Use of canthin-6-one for the preparation of a medicine intended for the treatment of trypanosomiasis according to claim 2, **characterised in that** the canthin-6-one is present in the form of a plant extract.

4. Use according to claim 3, **characterised in that** the canthin-6-one is present in the form of a plant extract selected from: *Ailanthus altissima, Brucea antidysenterica, Eurycoma harmandiana, Peganum nigellastrum, Zanthoxylum elephantiasis and Zanthoxylum chiloperone*.

5. Use according to claim 4, **characterised in that** the canthin-6-one is present in the form of an extract of *Zanthoxylum chiloperone var. angustifolium*.

6. Use according to any one of claims 1 to 5 for the preparation of a medicine intended for the treatment of trypanosomiasis in its chronic phase and its acute phase.

7. Use according to any one of claims 1 to 5 for the preparation of a medicine intended for the treatment of Chagas' disease.

8. Use according to any one of the preceding claims 1 to 6, **characterised in that** it is intended for the treatment of trypanosomiasis caused by the agent *Trypanosoma brucei.*

9. Use according to any one of the preceding claims 1 to 7, **characterised in that** it is intended for the treatment of trypanosomiasis caused by the agent *Trypanosoma cruzi.*

10. Use according to claim 5, **characterised in that** the plant extract containing the canthin-6-one is obtained by a process comprising a first stage which consists in grinding the dried bark of the trunk of *Zanthoxylum chiloperone var*. *angustifolium* and then in treating the bark with an aqueous alkaline solution.

11. Use according to claim 10, **characterised in that** the plant extract containing the canthin-6-one is obtained by a process comprising a second stage that consists of extraction by means of a chlorinated organic solvent.

12. Use according to any one of the preceding claims 1 to 11, **characterised in that** the medicine is intended to be administered in a dose between 0.01 and 100 mg/kg/d of formula (I) compound, preferably between 0.1 and 50 mg/kg/d, even more preferably between 1 and 20 mg/kg/d.

13. Use according to any one of the preceding claims, **characterised in that** the medicine is intended to be administered orally.

14. A compound in accordance with formula (I): 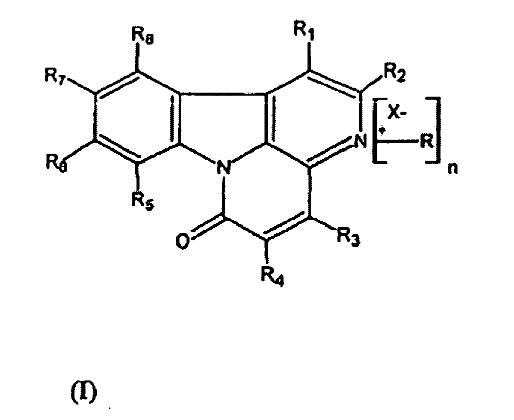 in which R₁, R₂, R₃, R₄, R₅, R₆, R₇, R₈ represent independently of one another:
○ a hydrogen atom,
○ a C₁-C₁₂ alkyl group that is linear, branched or cyclic, saturated or unsaturated,
• a halogen atom selected from chlorine, fluorine, bromine and iodine,
• a C₁-C₁₂ alkyl halide group, of which the alkyl chain can be linear, branched or cyclic, saturated or unsaturated, and the halogen atom or atoms is or are selected from fluorine, chlorine, bromine and iodine,
• a hydroxyl function
• an -NO nitro function
• a -CN cyano function
• an -SH function
• a -COOH carboxylic acid function
• a -CONH₂ amide function
• an -NH₂ amine function
• a C₁-C₁₂ alkoxy function, in which the alkyl group can be linear, branched or cyclic, saturated or unsaturated,
• a C₁-C₁₂ alkyl ester function, in which the alkyl group can be linear, branched or cyclic, saturated or unsaturated,
• a secondary or tertiary alkylamide function, in which the C₁-C₁₂ alkyl group or groups can be linear, branched or cyclic, saturated or unsaturated,
• a secondary or tertiary alkylamine function, in which the C₁-C₁₂ alkyl group or groups can be linear, branched or cyclic, saturated or unsaturated,
• a C₁-C₁₂ alkylthio function, in which the alkyl group can be linear, branched or cyclic, saturated or unsaturated,
• a C₂-C₆ heterocyclic group comprising 1 to 4 heteroatoms selected from sulphur, nitrogen and oxygen,
• an -SO₂-NR'R" group or an -NR'-SO₂R" group, in which R' and R" represent independently of one another a C₁-C₁₂ alkyl group that is linear, branched or cyclic, saturated or unsaturated;
n represents 0 or 1;
R represents a C₁-C₁₂ alkyl group that is linear, branched or cyclic, saturated or unsaturated,
X represents an anion that can be selected from inorganic or organic anions, it being understood that:
○ one at least of R₁, R₂, R₃, R₄, R₅, R₆, R₇ and R₈ is different from H or else n=1, and
○ when n = 0, R₂ = R₃ = R₄ = R₅ = R₆ = R₇ = H and R₈ = OCH₃, R₁ is different from -OH and from -OCH₃,
• when n = 0, R₁ = R₂ = R₃ = R₅ = R₆ = R₇ = R₈ = H, R₄ is different from -OCH₃,
• when n = 0, R₁ = R₂ = R₃ = R₄ = R₅ = R₇ = R₈ = H, R₆ is different from -OH and from -OCH₃,
• when n = 0, R₁ = R₂ = R₃ = R₄ = R₅ = R₈ = H, (R₆, R₇) is different from (-OCH₃, -OCH₃),
• when n = 0, R₂ = R₃ = R₄ = R₅ = R₆ = R₇ = R₈ = H, R₁ is different from -OCH₃,
• when n = 0, R₁ = R₂ = R₃ = R₄ = R₅ = R₆ = R₈ = H, R₇ is different from -OH,
and
R₇ is different from -OCH₃,
• when n = 0, R₂ = R₃ = R₄ = R₅ = R₆ = R₇ = H and R₁ = -OCH₃, R₈ is different from -OH,
• when n = 1, X = Cl, R = CH₃, R₁ = R₂ = R₅
= R₆ = R₇ = R₈ = H, and R₃ = -OCH₃ , R₄ is different from -OH.

15. A compound according to claim 14, **characterised in that** X- is chosen from: the Cl⁻ ion, the Br- ion, the I⁻ ion, the S⁻ ion, the PO₃⁻ ion, the NO₃⁻ ion, the acetate ion, the oxalate ion, the tartrate ion, the succinate ion, the maleate ion, the fumarate ion, the gluconate ion, the citrate ion, the malate ion, the ascorbate ion, the benzoate ion.

16. A compound according to claim 14 or claim 15, **characterised in that** one or more of the following conditions are fulfilled:
- R₃ represents an NH₂ group or a C₁-C₁₂ alkylamine group or a C₁-C₁₂ alkylamide group or a C₂-C₆ heterocylic group comprising at least one amine function;
- R₄ represents a hydroxyl group or a C₁-C₁₂ alkoxy group;
- R₁ = R₂ = R₅ = R₆ = R₇ = R₈ = H.

17. A compound according to any one of claims 14 to 16, **characterised in that** one or more of the following conditions are fulfilled:
- R₃ represents an NH₂ group or a C₁-C₆ alkylamine group or a C₁-C₆ alkylamide group or a C₂-C₆ heterocylic group comprising at least one amine function;
- R₄ represents a hydroxyl group or a C₁-C₆ alkoxy group;
- R₁ = R₂ = R₅ = R₆ = R₇ = R₈ = H.

18. A compound according to any one of claims 14 to 17, **characterised in that** one or more of the following conditions are fulfilled:
- R₃ represents an NH₂ group;
- R₄ represents an OCH₃ group;
- R₁ = R₂ = R₅ = R₆ = R₇ = R₈ = H.

19. A compound according to any one of claims 14 to 18, **characterised in that** R₁ = R₂ = R₃ = R₄ = R₅ = R₆ = R₇ = R₈ = H and n = 1, R is a C₁-C₆ alkyl group.

20. A compound according to any one of claims 14 to 18, **characterised in that** it is selected from:
- 4-aminocanthin-6-one;
- N-methylcanthin-6-one iodide.

21. A medicine, **characterised in that** it comprises a compound according to any one of claims 14 to 20 in a pharmaceutically acceptable base.

## Patentansprüche

1. Verwendung einer Verbindung gemäß Formel (I): 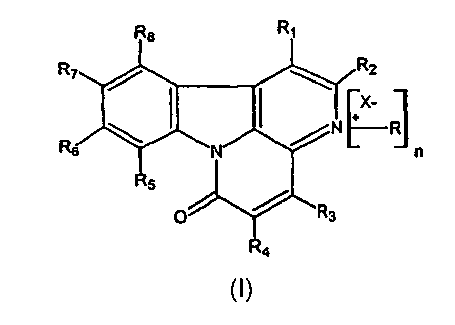 zur Herstellung eines Medikaments zur Behandlung einer Trypanosomiasis, worin R₁, R₂, R₃, R₄, R₅, R₆, R₇, R₈ unabhängig voneinander sind:
• ein Wasserstoffatom,
• eine lineare, verzweigte oder zyklische, gesättigte oder ungesättigte C₁-C₁₂-Alkylgruppe,
• ein Halogenatom ausgewählt aus Chlor, Fluor, Brom und Iod,
• eine C₁-C₁₂-Halogenalkylgruppe, deren Alkylkette linear, verzweigt oder zyklisch, gesättigt oder ungesättigt sein kann, und das oder die Halogenatome aus Chlor, Fluor, Brom oder Iod ausgewählt ist oder sind,
• eine Hydroxyfunktion,
• eine Nitrofunktion -NO,
• eine Cyanfunktion -CN,
• eine Funktion -SH,
• eine Carbonsäurefunktion -COOH,
• eine Amidfunktion -CONH₂,
• eine Aminfunktion -NH₂,
• eine C₁-C₁₂-Alkoxyfunktion, worin die Alkylgruppe linear, verzweigt oder zyklisch, gesättigt oder ungesättigt sein kann,
• eine C₁-C₁₂-Alkylesterfunktion, worin die Alkylgruppe linear, verzweigt oder zyklisch, gesättigt oder ungesättigt sein kann,
• eine sekundäre oder tertiäre C₁-C₁₂-Alkylamidfunktion, worin die Alkylgruppe(n) linear, verzweigt oder zyklisch, gesättigt oder ungesättigt sein kann oder können,
• eine sekundäre oder tertiäre C₁-C₁₂-Alkylaminfunktion, worin die Alkylgruppe(n) linear, verzweigt oder zyklisch, gesättigt oder ungesättigt sein kann oder können,
• eine C₁-C₁₂-Alkylthiolfunktion, worin die Alkylgruppe linear, verzweigt oder zyklisch, gesättigt oder ungesättigt sein kann,
• eine C₂-C₆ heterozyklische Gruppe, enthaltend 1 bis 4 Heteroatome, die aus Schwefel, Stickstoff und Sauerstoff ausgewählt sind,
• eine -SO₂-NR'R"-Gruppe oder eine -NR'-SO₂-R"-Gruppe, worin R' und R" unabhängig voneinander eine lineare, verzweigte oder zyklische, gesättigte oder ungesättigte C₁-C₁₂-Alkylgruppe sind,
wobei
• n 0 oder 1 ist,
• R ein lineare, verzweigte oder zyklische, gesättigte oder ungesättigte C₁-C₁₂-Alkylgruppe ist,
• X- ein Anion ist, das aus anorganischen oder organischen Anionen ausgewählt sein kann.

2. Verwendung nach Anspruch 1, **dadurch gekennzeichnet, dass** die Verbindung der Formel (I) Canthin-6-on ist.

3. Verwendung von Canthin-6-on zur Herstellung eines Medikamentes zur Behandlung einer Trypanosomiasis nach Anspruch 2, **dadurch gekennzeichnet, dass** das Canthin-6-on in Form eines Pflanzenextraktes vorliegt.

4. Verwendung nach Anspruch 3, **dadurch gekennzeichnet, dass** das Canthin-6-on in Form eines Extraktes einer Pflanze vorliegt, ausgewählt aus: *Ailanthus altissima, Brucea antidysenterica, Eurycoma harmandiana, Peganum nigellastrum, Zanthoxylum elephantiasis and Zanthoxylan chiloperone.*

5. Verwendung nach Anspruch 4, **dadurch gekennzeichnet, dass** das Canthin-6-on in Form eines Extraktes aus *Zanthoxylum chilperone* var. *angustifolium* vorliegt.

6. Verwendung nach einem der Ansprüche 1 bis 5 zur Herstellung eines Medikaments zur Behandlung einer Trypanosomiasis in ihrer chronischen oder akuten Phase.

7. Verwendung nach einem der Ansprüche 1 bis 5 zur Herstellung eines Medikamentes zur Behandlung der Chagas-Krankheit.

8. Verwendung nach einem der Ansprüche 1 bis 6, **dadurch gekennzeichnet, dass** sie zur Behandlung einer von dem Erreger *Trypanosoma brucei* verursachten Trypanosomiasis ist.

9. Verwendung nach einem der Ansprüche 1 bis 7, **dadurch gekennzeichnet, dass** sie zur Behandlung einer von dem Erreger *Trypanosoma cruzi* verursachten Trypanosomiasis ist.

10. Verwendung nach Anspruch 5, **dadurch gekennzeichnet, dass** der Canthin-6-on enthaltende Pflanzenextrakt mittels eines Verfahrens hergestellt wird, umfassend einen ersten Schritt bestehend aus Vermahlen der getrockneten Stammrinde des *Zanthoxylum chilperone* var. *angustifolium* und deren anschließende Behandlung mit einer wässrigen alkalischen Lösung.

11. Verwendung nach Anspruch 10, **dadurch gekennzeichnet, dass** der Canthin-6-on enthaltende Pflanzenextrakt mittels eines Verfahrens hergestellt wird, umfassend einen zweiten Schritt bestehend aus einer Extraktion mit einem chlorierten organischen Lösungsmittel.

12. Verwendung nach einem der vorherigen Ansprüche 1 bis 11, **dadurch gekennzeichnet, dass** das Medikament zur Verabreichung in einer Dosis zwischen 0,01 und 100 mg/kg/Tag der Verbindung der Formel (I), bevorzugt zwischen 0,1 und 50 mg/kg/Tag, und besonders bevorzugt zwischen 1 und 20 mg/kg/Tag, bestimmt ist.

13. Verwendung nach einem der vorherigen Ansprüche, **dadurch gekennzeichnet, dass** das Medikament für die orale Verabreichung ist.

14. Verbindung gemäß Formel (I): 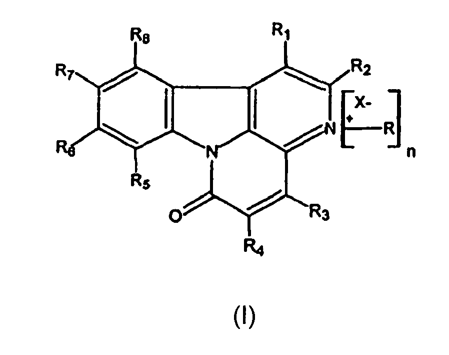 worin R₁, R₂, R₃, R₄, R₅, R₆, R₇, R₈ unabhängig voneinander sind:
• ein Wasserstoffatom,
• eine lineare, verzweigte oder zyklische, gesättigte oder ungesättigte C₁-C₁₂-Alkylgruppe,
• ein Halogenatom ausgewählt aus Chlor, Fluor, Brom und Iod,
• eine C₁-C₁₂-Halogenalkylgruppe, deren Alkylkette linear, verzweigt oder zyklisch, gesättigt oder ungesättigt sein kann, und das oder die Halogenatome aus Chlor, Fluor, Brom oder Iod ausgewählt ist oder sind,
• eine Hydroxyfunktion,
• eine Nitrofunktion -NO,
• eine Cyanfunktion -CN,
• eine Funktion -SH,
• eine Carbonsäurefunktion -COOH,
• eine Amidfunktion -CONH₂,
• eine Aminfunktion -NH₂,
• eine C₁-C₁₂-Alkoxyfunktion, worin die Alkylgruppe linear, verzweigt oder zyklisch, gesättigt oder ungesättigt sein kann,
• eine C₁-C₁₂-Alkylesterfunktion, worin die Alkylgruppe linear, verzweigt oder zyklisch, gesättigt oder ungesättigt sein kann
• eine sekundäre oder tertiäre C₁-C₁₂-Alkylamidfunktion, worin die Alkylgruppe(n) linear, verzweigt oder zyklisch, gesättigt oder ungesättigt sein kann oder können,
• eine sekundäre oder tertiäre C₁-C₁₂-Alkylaminfunktion, worin die Alkylgruppe(n) linear, verzweigt oder zyklisch, gesättigt oder ungesättigt sein kann oder können,
• eine C₁-C₁₂-Alkylthiolfunktion, worin die Alkylgruppe linear, verzweigt oder zyklisch, gesättigt oder ungesättigt sein kann,
• eine C₂-C₆ heterozyklische Gruppe, enthaltend 1 bis 4 Heteroatome, die aus Schwefel, Stickstoff und Sauerstoff ausgewählt sind,
• eine -SO₂-NR'R"-Gruppe oder einer -NR'-SO₂-R"-Gruppe, worin R' und R" unabhängig voneinander eine lineare, verzweigte oder zyklische, gesättigte oder ungesättigte C₁-C₁₂-Alkylgruppe sind,
wobei
• n 0 oder 1 ist,
• R ein lineare, verzweigte oder zyklische, gesättigte oder ungesättigte C₁-C₁₂-Alkylgruppe ist,
• X- ein Anion ist, das aus anorganischen oder organischen Anionen ausgewählt sein kann, mit der Einschränkung, dass:
• wenigstens einer von R₁, R₂, R₃, R₄, R₅, R₆, R₇ und R₈ verschieden von H ist oder andernfalls n = 1 ist, und
• wenn n = 0, R₂ = R₃ = R₄ = R₅ = R₆ = R₇ = H und R₈ = OCH₃, R₁ verschieden von -OH und -OCH₃ ist,
• wenn n = 0, R₁ = R₂ = R₃ = R₅ = R₆ = R₇ = R₈ = H, R₄ verschieden von -OCH₃ ist,
• wenn n=0, R₁ = R₂ = R₃ = R₄ = R₅ = R₇ = R₈ = H, R₆ verschieden von -OH und -OCH₃ ist,
• wenn n = 0, R₁ = R₂ = R₃ = R₄ = R₅ = R₈ = H, (R₆, R₇) verschieden von (-OCH₃, -OCH₃) ist,
• wenn n = 0, R₂ = R₃ = R₄ = R₅ = R₆ = R₇ = R₈ = H, R₁ verschieden von -OCH₃ ist,
• wenn n = 0, R₁ = R₂ = R₃ = R₄ = R₅ = R₆ = R₈ = H, R₇ verschieden von -OH ist und R₇ verschieden von -OCH₃ ist,
• wenn n = 0, R₂ = R₃ = R₄ = R₅ = R₆ = R₇ = H und R₁ = -OCH₃, R₈ verschieden von -OH ist,
• wenn n = 1, X = Cl, R = CH₃, R₁ = R₂ = R₅ = R₆ = R₇ = R₈ = H und R₃ = -OCH₃, R₄ verschieden von -OH ist.

15. Verbindung nach Anspruch 14, **dadurch gekennzeichnet, dass** X- ausgewählt ist aus: einem Cl⁻-Ion, einem Br⁻-Ion, einem I⁻-Ion, einem S⁻-Ion einem PO₃⁻-Ion, einem NO₃⁻-Ion, einem Acetat-Ion, einem Oxalat-Ion, einem Tartrat-Ion, einem Succinat-Ion, einem Maleat-Ion, einem Fumarat-Ion, einem Gluconat-Ion, einem Citrat-Ion, einem Malat-Ion, einem Ascorbat-Ion, einem Benzoat-Ion.

16. Verbindung nach Anspruch 14 oder Anspruch 15, **dadurch gekennzeichnet, dass** eine oder mehrere der folgenden Bedingungen erfüllt sind:
- R₃ ist eine NH₂-Gruppe oder eine C₁-C₁₂-Alkylamingruppe oder eine C₁-C₁₂-Alkylamidgruppe oder ein C₂-C₆-Heterozyklus, der wenigstens eine Aminfunktion umfasst;
- R₄ ist eine Hydroxygruppe oder eine C₁-C₁₂-Alkoxygruppe;
- R₁ = R₂ = R₅ = R₆ = R₇ = R₈ = H.

17. Verbindung nach einem der Ansprüche 14 bis 16, **dadurch gekennzeichnet, dass** eine oder mehrere der folgenden Bedingungen erfüllt sind:
- R₃ ist eine NH₂-Gruppe oder eine C₁-C₆-Alkylamingruppe oder eine C₁-C₆-Alkylamidgruppe oder ein C₂-C₆-Heterozyklus, der wenigstens eine Aminfunktion umfasst;
- R₄ ist eine Hydroxygruppe oder eine C₁-C₆-Alkoxygruppe;
- R₁ = R₂ = R₅ = R₆ = R₇ = R₈ = H.

18. Verbindung nach einem der Ansprüche 14 bis 17, **dadurch gekennzeichnet, dass** eine oder mehrere der folgenden Bedingungen erfüllt sind:
- R₃ ist eine NH₂-Gruppe;
- R₄ ist eine OCH₃-Gruppe;
- R₁ = R₂ = R₅ = R₆ = R₇ = R₈ = H.

19. Verbindung nach einem der Ansprüche 14 bis 18, **dadurch gekennzeichnet, dass** R₁ = R₂ = R₃ = R₄ = R₅ = R₆ = R₇ = R₈ = H, n = 1 und R eine C₁-C₆-Alkylgruppe ist.

20. Verbindung nach einem der Ansprüche 14 bis 18, **dadurch gekennzeichnet, dass** diese ausgewählt ist aus:
- 4-Amino-canthin-6-on;
- N-Methyl-canthin-6-on-iodid.

21. Medikament, **dadurch gekennzeichnet, dass** es eine Verbindung nach einem der Ansprüche 14 bis 20 in einem pharmazeutisch verträglichen Träger umfasst.
